(19) Europäisches Patentamt
European Patent Office
Office européen des brevets

(11) **EP 4 265 253 A1**

(12) **EUROPEAN PATENT APPLICATION**
published in accordance with Art. 153(4) EPC

(43) Date of publication:
**25.10.2023 Bulletin 2023/43**

(21) Application number: **21906480.5**

(22) Date of filing: **09.12.2021**

(51) International Patent Classification (IPC):
***A61K 31/4965*** *(2006.01)* ***A61K 9/08*** *(2006.01)*
***A61K 9/19*** *(2006.01)* ***A61K 47/04*** *(2006.01)*
***A61K 47/18*** *(2017.01)* ***A61K 47/20*** *(2006.01)*
***A61K 47/22*** *(2006.01)* ***A61P 31/12*** *(2006.01)*

(52) Cooperative Patent Classification (CPC):
**A61K 9/08; A61K 9/19; A61K 31/4965; A61K 47/02; A61K 47/18; A61K 47/20; A61K 47/22; A61P 31/12**

(86) International application number:
**PCT/JP2021/045287**

(87) International publication number:
**WO 2022/131117 (23.06.2022 Gazette 2022/25)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **18.12.2020 JP 2020210030**

(71) Applicant: **FUJIFILM Toyama Chemical Co., Ltd.**
**Chuo-ku**
**Tokyo 104-0031 (JP)**

(72) Inventor: **KAKITA Kosuke**
**Toyama-shi, Toyama 930-8508 (JP)**

(74) Representative: **Blodig, Wolfgang**
**Wächtershäuser & Hartz**
**Patentanwaltspartnerschaft mbB**
**Weinstrasse 8**
**80333 München (DE)**

(54) **PHARMACEUTICAL COMPOSITION**

(57) A pharmaceutical composition comprising Components (1) and (2) :
(1) 6-fluoro-3-hydroxy-2-pyrazinecarboxamide or a salt thereof; and
(2) a compound having a partial structure containing two heteroatoms separated by at least two carbon atoms, or a sulfite thereof.

EP 4 265 253 A1

## Description

[Technical Field]

The present invention relates to a pharmaceutical composition containing 6-fluoro-3-hydroxy-2-pyrazinecarboxamide (hereinafter may be referred to as "Compound A") and having improved stability.

[Background Art]

Compound A or a salt thereof has a superior antiviral activity and is useful as a therapeutic agent for viral infection (Patent Literature 1). Compound A has a low water solubility; however, sodium salts (Patent Literature 2) and meglumine salts (Patent Literature 3) of Compound A are known to have a relatively high solubility and be useful as a preparation for injection.

When Compound A is used as a liquid preparation, such as a preparation for injection or a syrup, improved stability of Compound A in an aqueous solution would be advantageous for long-term storage and stockpiling. However, such studies have not been conducted so far.

[Citation List]

[Patent Literature]

[Patent Literature 1]
International Publication No. WO 00/10569
[Patent Literature 2]
International Publication No. WO 2012/043700
[Patent Literature 3]
International Publication No. WO 2012/043696

[Summary of Invention]

[Technical Problem]

The object of the present invention is to provide a pharmaceutical composition with improved stability of Compound A in an aqueous solution.

[Solution to Problem]

As a result of extensive studies to achieve the above-mentioned object, the present inventors found that a pharmaceutical composition in which the above-mentioned object was achieved could be provided by using Compound A in combination with a specific stabilizing component, and thus the present invention was accomplished.

One aspect of the present invention is described below.

<1> A pharmaceutical composition comprising Components (1) and (2) :

(1) 6-fluoro-3-hydroxy-2-pyrazinecarboxamide or a salt thereof; and
(2) a compound having a partial structure containing two heteroatoms separated by at least two carbon atoms, or a sulfite thereof.

<2> The pharmaceutical composition according to <1>, wherein Component (2) is at least one of the following Components (2-1) to (2-6):

(2-1) a hydroxyalkylamine;
(2-2) a heterocyclic amine;
(2-3) an $\alpha$-amino acid with an isoelectric point of 10 or lower;
(2-4) an aminocarboxylic acid chelating agent;
(2-5) a $\beta$-aminosulfonic acid; and

(2-6) a sulfite.

<3> The pharmaceutical composition according to <2>, wherein the hydroxyalkylamine of Component (2-1) is represented by the following general formula [1] or [2]:

[Formula 1]

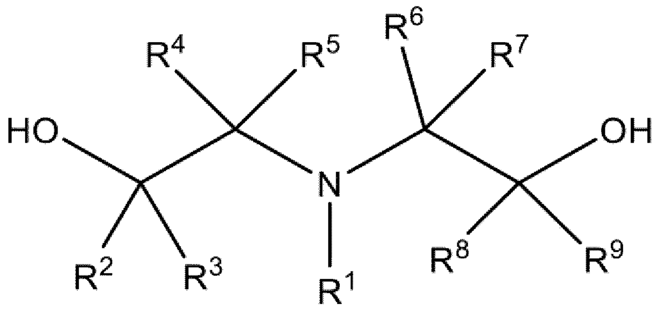

[1]

wherein $R^1$ represents a hydrogen atom or a $C_{1-3}$ alkyl group, wherein the $C_{1-3}$ alkyl group may have a hydroxy group or a carboxy group as a substituent group, and $R^2$ to $R^9$, which are identical to or different from each other, represent a hydrogen atom or a $C_{1-3}$ alkyl group.

[Formula 2]

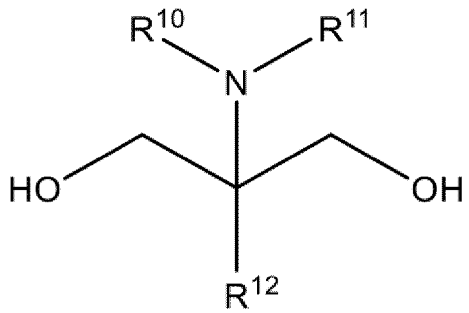

[2]

wherein $R^{10}$ and $R^{11}$, which are identical to or different from each other, represent a hydrogen atom or a $C_{1-3}$ alkyl group, wherein the $C_{1-3}$ alkyl group may have a hydroxy group or a carboxy group as a substituent group, and $R^{12}$ represents a hydrogen atom or a $C_{1-3}$ alkyl group, wherein the $C_{1-3}$ alkyl group may have a hydroxy group as a substituent group.

<4> The pharmaceutical composition according to <2>, wherein the hydroxyalkylamine of Component (2-1) is selected from the following compound group: a compound group consisting of trometamol, diethanolamine, triethanolamine, diisopropanolamine, N-ethyldiethanolamine, bicine, and tricine.

<5> The pharmaceutical composition according to any of <2> to <4>, wherein the heterocyclic amine of Component (2-2) is DABCO.

<6> The pharmaceutical composition according to any of <2> to <5>, wherein the α-amino acid with an isoelectric point of 10 or lower of Component (2-3) is selected from the following compound group:

serine, threonine, histidine, valine, leucine, glutamic acid, glutamine, cysteine, phenylalanine, aspartic acid, asparagine, glycine, alanine, and salts of these amino acids.

<7> The pharmaceutical composition according to any of <2> to <6>, wherein the aminocarboxylic acid chelating agent of Component (2-4) is EDTA.

<8> The pharmaceutical composition according to any of <2> to <7>, wherein the β-aminosulfonic acid of Component

(2-5) is taurine or HEPES.

<9> The pharmaceutical composition according to any of <2> to <8>, wherein the sulfite of Component (2-6) is sodium pyrosulfite.

<10> The pharmaceutical composition according to any of <1> to <9>, comprising 0.05 to 5 equivalents of Component (2) to 6-fluoro-3-hydroxy-2-pyrazinecarboxamide or a salt thereof.

<11> The pharmaceutical composition according to any of <1> to <10>, which further comprises Component (3) and is an aqueous solution:

(3) water.

<12> The pharmaceutical composition according to <11>, wherein the concentration of Component (2) is 0.1 to 5% w/v.

<13> The pharmaceutical composition according to <11> or <12>, wherein the aqueous solution is pH 6.7 to 12.5.

<14> The pharmaceutical composition according to <1> to <10>, which is a lyophilized preparation.

[Advantageous Effect of Invention]

According to the present invention, a pharmaceutical composition with improved stability of Compound A in an aqueous solution is provided.

[Description of Embodiments]

In the present specification, the range of numerical values expressed using "to" means a range inclusive of numerical values written before and after "to" as a minimum value and a maximum value, respectively. In one aspect, the value itself of one of a minimum value and a maximum value or both may be excluded (that is, the range can mean "more than x" and "less than x" instead of "x or more" and "x or less").

In the present specification, when a plurality of substances corresponding to each component exist in the composition, the amount of each component in the composition means the total amount of the plurality of substances in the composition, unless otherwise specified.

In the present specification, each term has the following meaning, unless otherwise specified.

The term "$C_{1-3}$ alkyl group" means, for example, a linear or branched chain alkyl group having one to three carbon atoms, such as methyl group, ethyl group, propyl group, or isopropyl group.

<Compound A>

In the present invention, Compound A (6-fluoro-3-hydroxy-2-pyrazinecarboxamide) or a salt thereof is used as an active ingredient. A salt can be any pharmaceutically acceptable salt and is preferably a sodium salt or a meglumine salt.

If Compound A has isomers (e.g., an optical isomer, a geometric isomer, and a tautomer), the present invention encompasses all the isomers, as well as hydrates, solvates, and all crystalline forms.

stabilizing component>

A stabilizing component used in the present invention can be any component as long as the component can improve the stability of Compound A in an aqueous solution when it is used in combination with Compound A. The expression "improvement of stability" means that, after a predetermined time passes, the residual rate of Compound A is higher, or the degree of coloring in an aqueous solution is lower as compared with when a stabilizing component is not added. Specifically, when Compound A is left in a state of an aqueous solution at 70°C for seven days (this condition is considered to be equivalent to being left at room temperature for one year), the residual rate of Compound A is preferably 93% or higher, more preferably 95% or higher, yet more preferably 97% or higher in view of the residual rate of Compound A, and the color preferably does not turn black, preferably remaining pale yellow, yellow, pale orange, orange, and brown in this order in view of the degree of coloring of an aqueous solution. A stabilizing component is preferably a compound that has a partial structure containing two heteroatoms separated by at least two carbon atoms. The compound is preferably a low molecular compound having an amino group. The partial structure preferably has two carbon atoms which are preferably saturated, and it is preferred that one heteroatom is nitrogen, and the other is oxygen or sulfur. Particularly preferred examples of the partial structure include N-C-C-O and N-C-C-S. Specific examples of a preferred compound include (1) a hydroxyalkylamine, (2) a heterocyclic amine, (3) an $\alpha$-amino acid with an isoelectric point of 10 or lower, (4) an aminocarboxylic acid chelating agent, and (5) a $\beta$-aminosulfonic acid. Another aspect of a preferred stabilizing component includes (6) a sulfite. Among the above-mentioned (1) to (6), (1) a hydroxyalkylamine, (4) an aminocarboxylic acid chelating agent, and (3) an $\alpha$-amino acid with an isoelectric point of 10 or lower are preferred, and (1) a hydroxyalkylamine is more preferred.

One or more stabilizing components can be used in combination.

<Hydroxyalkylamine>

A hydroxyalkylamine is an amine compound having a hydroxyalkyl group (an alkyl group of two or more carbon atoms having a hydroxy group as a substituent group), or a salt thereof. Examples thereof include trometamol, diethanolamine, triethanolamine, diisopropanolamine, triisopropanolamine, N-ethyldiethanolamine, N,N-bis(2-hydroxyethyl) glycine (bicine), and N-[tris(hydroxymethyl)methyl]glycine (tricine). Although complete details are not clear, it is inferred that an ethanolamine structure existing in a hydroxyalkylamine contributes greatly to stabilization. Therefore, a hydroxyalkylamine is preferably a hydroxyalkylamine having an ethanolamine structure.

The structure of a hydroxyalkylamine is not particularly limited, but a hydroxyalkylamine represented by general formula [1] is preferred. In the hydroxyalkylamine represented by general formula [1],

$R^1$ is preferably a hydrogen atom, an ethyl group, a carboxymethyl group, or a hydroxyethyl group, more preferably a hydrogen atom; and

$R^2$ to $R^9$, which are identical to or different from each other, are preferably a hydrogen atom or a methyl group.

An amine represented by general formula [1] is preferably diethanolamine, triethanolamine, diisopropanolamine, triisopropanolamine, N-ethyldiethanolamine, or bicine.

As another aspect, a hydroxyalkylamine is preferably a hydroxyalkylamine represented by general formula [2]. In the hydroxyalkylamine represented by general formula [2],

> $R^{10}$ to $R^{11}$, which are identical to or different from each other, are preferably a hydrogen atom or a carboxymethyl group; and
>
> $R^{12}$ is preferably a hydroxyethyl group.

An amine represented by general formula [2] is preferably trometamol or tricine.

<Heterocyclic amine>

A heterocyclic amine is a compound containing at least one heterocyclic ring, having at least one amine functional group (containing nitrogen), and having a partial structure containing heteroatoms separated by at least two carbon atoms, or a salt thereof. Examples thereof include piperazine, morpholine, triazine, and 1,4-diazabicyclo[2.2.2]octane (DABCO). A heterocyclic amine is preferably DABCO.

<α-Amino acid with isoelectric point of 10 or lower>

An α-amino acid with an isoelectric point of 10 or lower is a natural or unnatural α-amino acid with an isoelectric point of 10 or lower, or a salt thereof. Examples thereof include isoleucine, methionine, lysine, histidine, tyrosine, tryptophan, proline, serine, threonine, histidine, valine, leucine, glutamic acid, glutamine, cysteine, phenylalanine, aspartic acid, asparagine, glycine, and alanine. An α-amino acid with an isoelectric point of 10 or lower is preferably serine, threonine, histidine, valine, leucine, glutamic acid, glutamine, cysteine, phenylalanine, aspartic acid, asparagine, glycine, or alanine.

<Aminocarboxylic acid chelating agent>

An aminocarboxylic acid chelating agent is a chelating agent that has a nitrogen atom and a carboxy group as well as a partial structure containing heteroatoms separated by at least two carbon atoms. Examples thereof include ethylenediamine tetraacetic acid (EDTA), glycol ether diamine tetraacetic acid (EGTA), nitrilotriacetic acid (NTA), diethylene triamine pentaacetic acid (DTPA), hydroxyethyl ethylenediamine tetraacetic acid (HEDTA), hydroxyethyl ethylenediamine triacetic acid (HEDTA), methylglycinediacetic acid (MGDA), L-glutamic acid diacetic acid (GLDA), aspartic acid diacetic acid (ASDA), ethylenediamine succinic acid (EDDS), hydroxyiminodisuccinic acid (HIDS), iminodisuccinic acid (IDS), and salts of the above-mentioned compounds. An aminocarboxylic acid chelating agent is preferably EDTA.

<β-Aminosulfonic acid>

A β-aminosulfonic acid is a natural or unnatural β-aminosulfonic acid or a salt thereof. Examples thereof include taurine, 4-(2-hydroxyethyl)-1-piperazineethanesulfonic acid (HEPES), and 2-(methylamino)ethanesulfonic acid. A β-aminosulfonic acid is preferably taurine or HEPES.

<Sulfite>

A sulfite is a salt that is dissociated to sulfite ions in a solution, and examples thereof include a sulfite in the narrow sense, a hydrogen sulfite, and a pyrosulfite. Examples of a counter cation include an alkali metal ion and an alkaline earth metal ion. Examples of a sulfite include sodium sulfite, dried sodium sulfite, potassium sulfite, calcium sulfite, sodium hydrogen sulfite, potassium hydrogen sulfite, ammonium hydrogen sulfite, sodium pyrosulfite, and potassium pyrosulfite. A sulfite is preferably sodium pyrosulfite.

The amount of a stabilizing component used is not particularly limited as long as the amount can improve stability of Compound A in an aqueous solution. The lower limit is preferably 0.01, 0.02, 0.04, 0.05, or 0.1 equivalents to Compound A, and the upper limit is preferably 10, 5, 2, 1, 0.5, or 0.2 equivalents, particularly preferably 0.1 to 0.2 equivalents.

A stabilizing component is manufactured by methods known per se or by suitably using these methods in combination, or any commercially available product can be used.

<Water>

Water used in the present invention is not particularly limited as long as it is suitable for manufacture of a pharmaceutical composition and is preferably purified water, water of a grade equivalent to or higher than purified water, or water for injection.

<Pharmaceutical composition>

In addition to a composition obtained by dissolving Compound A together with a stabilizing component in water (i.e., a liquid preparation), the pharmaceutical composition of the present invention includes a composition obtained by lyophilizing the liquid preparation (i.e., a lyophilized preparation). Both a liquid preparation and a lyophilized preparation can be used as a preparation for injection or an oral preparation. When Compound A is used as a liquid preparation, the concentration of Compound A is preferably 100 mg/mL or higher, more preferably 200 mg/mL or higher. As a mass volume percentage (% w/v) concentration, the lower limit of the concentration of a stabilizing component is preferably 0.01, 0.02, 0.04, 0.1, or 0.3% w/v, and the upper limit is preferably 10, 5, or 2% w/v. The concentration is particularly preferably in a range of 0.3 to 2% w/v. The pH of an aqueous solution is preferably neutral to alkaline, more preferably weakly alkaline. Specifically, pH is preferably 6.7 to 12.5, more preferably 8 to 10, yet more preferably 8.5 to 9.5.

[Method for manufacturing liquid preparation]

Step (1):

An aqueous solution of Compound A is prepared by dissolving Compound A or a salt thereof together with a stabilizing component in water. The expression "together with a stabilizing component" means that Compound A and a stabilizing component may be added to water simultaneously, or either component may be added to water first, followed by addition of the remaining component.

Step (2) :

The aqueous solution obtained in Step (1) is filled into a drug product container (e.g., a vial), and then the container is sealed to obtain a liquid preparation. Step (1) and Step (2) may be performed simultaneously. That is, a liquid preparation may be obtained by directly preparing an aqueous solution of Compound A in a drug product container.

[Method for manufacturing lyophilized preparation]

Step (1):

Compound A is dissolved together with a stabilizing component in water to prepare an aqueous solution of Compound A. The expression "together with a stabilizing component" means that Compound A and a stabilizing component may be added to water simultaneously, or either component may be added to water first, followed by addition of the remaining component.

Step (2):

The aqueous solution obtained in Step (1) is filled into a drug product container (e.g., a vial). Step (1) and Step (2) may be performed simultaneously. That is, an aqueous solution of Compound A may be directly prepared in a drug product container.

Step (3) :

The aqueous solution obtained in Step (1) is lyophilized by a usual lyophilization method. The order of Step (2) and Step (3) is not limited. That is, these steps are performed as follows:

Step (3) following Step (2):

The aqueous solution filled into a drug product container at Step (2) is lyophilized at Step (3).

Step (2) following Step (3):

The aqueous solution obtained in Step (1) is lyophilized at Step (3), and then the lyophilized preparation is filled into a drug product container at Step (2).

[Administration of pharmaceutical composition]

The administration method, dose, and number of doses of the pharmaceutical composition of the present invention can be suitably selected depending on patient's age, body weight, and symptom. Usually, the amount that can exhibit a drug effect may be divided into one to several doses per day and intramuscularly or intravenously injected or orally administered.

<Other additives>

To the pharmaceutical composition of the present invention, an osmotic pressure regulator, a pH regulator, a buffer, a surfactant, a soothing agent, a sweetener and/or a preservative, and the like which are commonly used may be added as necessary.

Examples of an osmotic pressure regulator include sodium chloride, glycerin, and propylene glycol.

Examples of a pH regulator and/or a buffer include acids such as hydrochloric acid, phosphoric acid, sulfuric acid, methanesulfonic acid, acetic acid, lactic acid, maleic acid, citric acid, tartaric acid, ascorbic acid, and benzoic acid; salts such as sodium hydrogen carbonate, sodium carbonate, sodium dihydrogen phosphate, potassium dihydrogen phosphate, disodium hydrogen phosphate, dipotassium hydrogen phosphorate, trisodium phosphate, disodium citrate, sodium deoxycholate, and sodium sulfite; and bases such as sodium hydroxide, trometamol, monoethanolamine, diethanolamine, triethanolamine, L-arginine, and L-lysine.

Examples of a surfactant include a sorbitan fatty acid ester, polyoxyethylene hydrogenated castor oil, polyoxyethylene sorbitan monolaurate, polyoxyethylene polyoxypropylene glycol, and polysorbate.

Examples of a soothing agent include lidocaine, procaine, meprylcaine, and benzyl alcohol.

Examples of a preservative include cresol, phenol, methyl paraoxybenzoate, ethyl paraoxybenzoate, benzalkonium chloride, and benzethonium chloride.

Examples of a sweetener include fructose, glucose, liquid sugar, honey, erythritol, xylitol, saccharin, sucralose, aspartame, and acesulfame potassium.

[Examples]

The present invention is described in more detail below using the following Test Examples and Examples. However, the present invention is not limited to these examples.

Test Example 1: Residual rate

To examine the stabilizing effect of a stabilizing component, the residual rate of Compound A was determined. Specifically, Compound A was suspended in water, 0.14 to 0.16 equivalents of a stabilizing component and sodium hydroxide were added to dissolve Compound A, the mixture was adjusted to pH 8.9 to 9.1 with sodium hydroxide or hydrochloric acid as necessary and then filtered through a filter with a pore size of 0.45 μm, and the filtrate was left at

70°C for three or seven days. Subsequently, the drug concentration in each filtrate was measured, and the residual rate was calculated by a method represented by Equation 1. The results are shown in Table 1.

Equation 1

$$\text{Residual rate [\%]} = \text{drug concentration after leaving at 70°C} / \text{drug concentration at manufacture} \times 100$$

Test Example 2: Appearance

To examine the stabilizing effect of a stabilizing component, the degree of coloring was determined. Specifically, Compound A was suspended in water, 0.14 to 0.16 equivalents of a stabilizing component and sodium hydroxide were added to dissolve Compound A, the mixture was adjusted to pH 8.9 to 9.1 with sodium hydroxide or hydrochloric acid as necessary and then filtered through a filter with a pore size of 0.45 μm, and the filtrate was left at 70°C for seven days (it should be noted that being left at 70°C for seven days is considered equivalent to being left at room temperature for one year). Subsequently, the color tone of each filtrate was observed visually. It should be noted that an aqueous solution of Compound A is pale yellow at the time of manufacture and turns yellow, pale orange, orange, brown, and eventually black as coloring proceeds. The results are shown in Table 1.

[Table 1]

| Example number | Stabilizing component | | | pH at manufacture | Number of days of storage at 70°C (days) | Appearance | Residual rate (%) |
|---|---|---|---|---|---|---|---|
| | Name | Number of equivalents | Concentration [% w/v] | | | | |
| Example 1 | Trometamol | 0.15 | 0.6 | 9.0 | 7 | Yellow | 97 |
| Example 2 | Diethanolamine | 0.16 | 0.5 | 9.0 | 7 | Pale orange | 97 |
| Example 3 | Triethanolamine | 0.15 | 0.7 | 9.0 | 7 | Pale orange | 98 |
| Example 4 | Diisopropanolamine | 0.15 | 0.7 | 9.0 | 7 | Yellow | 99 |
| Example 5 | Triisopropanolamine | 0.16 | 1.0 | 9.0 | 7 | Pale orange | 96 |
| Example 6 | N-Ethyldiethanolamine | 0.15 | 0.6 | 9.0 | 7 | Yellow | 97 |
| Example 7 | bicine | 0.15 | 0.8 | 9.0 | 7 | Yellow | 96 |
| Example 8 | tricine | 0.15 | 0.8 | 9.0 | 7 | Yellow | 95 |
| Example 9 | DABCO | 0.16 | 0.6 | 9.0 | 7 | Yellow | 97 |
| Example 10 | Serine | 0.15 | 0.5 | 9.0 | 7 | Yellow | 97 |
| Example 11 | Threonine | 0.15 | 0.6 | 9.0 | 7 | Yellow | 97 |
| Example 12 | Histidine | 0.16 | 0.8 | 9.0 | 7 | Orange | 95 |
| Example 13 | Valine | 0.15 | 0.6 | 9.0 | 7 | Pale orange | 99 |
| Example 14 | Leucine | 0.15 | 0.6 | 9.0 | 7 | Orange | 101 |
| Example 15 | Glutamic acid | 0.15 | 0.7 | 9.0 | 7 | Pale orange | 96 |
| Example 16 | Asparagine | 0.15 | 0.7 | 9.0 | 7 | Pale orange | 96 |
| Example 17 | Glycine | 0.15 | 0.4 | 9.0 | 7 | Brown | 97 |
| Example 18 | Alanine | 0.15 | 0.4 | 9.0 | 7 | Brown | 90 |
| Example 19 | EDTA | 0.15 | 1.8 | 9.0 | 7 | Pale Yellow | 97 |
| Example 20 | Sodium pyrosulfite | 0.15 | 0.9 | 9.0 | 7 | Pale orange | 93 |
| Example 21 | HEPES | 0.15 | 1.2 | 9.0 | 7 | Pale orange | 96 |
| Example 22 | Aspartic acid | 0.15 | 0.6 | 9.0 | 3 | Yellow | 99 |

(continued)

| Example number | Stabilizing component | | | pH at manufacture | Number of days of storage at 70°C (days) | Appearance | Residual rate (%) |
|---|---|---|---|---|---|---|---|
| | Name | Number of equivalents | Concentration [% w/v] | | | | |
| Comparative Example 1 | Sodium hydroxide | - | - | 8.9 | 7 | Black | 87 |
| Comparative Example 2 | Meglumine | 0.15 | 1.0 | 9.0 | 7 | Black | 92 |

Example 1

To a suspension of 503 mg of Compound A in 5 mL of water, 58 mg of trometamol was added, and the mixture was adjusted to pH 9.0 and diluted with water to make 10 mL. It was confirmed that pH remained unchanged after dilution. The residual rate was measured by the method shown in Test Example 1, and the appearance was examined by the method shown in Test Example 2.

Example 2

To a suspension of 500 mg of Compound A in 5 mL of water, 52 mg of diethanolamine was added, and the mixture was adjusted to pH 9.0 and diluted with water to make 10 mL. It was confirmed that pH remained unchanged after dilution. The residual rate was measured by the method shown in Test Example 1, and the appearance was examined by the method shown in Test Example 2.

Example 3

To a suspension of 500 mg of Compound A in 5 mL of water, 72 mg of triethanolamine was added, and the mixture was adjusted to pH 9.0 and diluted with water to make 10 mL. It was confirmed that pH remained unchanged after dilution. The residual rate was measured by the method shown in Test Example 1, and the appearance was examined by the method shown in Test Example 2.

Example 4

To a suspension of 501 mg of Compound A in 5 mL of water, 65 mg of diisopropanolamine was added, and the mixture was adjusted to pH 9.0 and diluted with water to make 10 mL. It was confirmed that pH remained unchanged after dilution. The residual rate was measured by the method shown in Test Example 1, and the appearance was examined by the method shown in Test Example 2.

Example 5

To a suspension of 496 mg of Compound A in 5 mL of water, 97 mg of triisopropanolamine was added, and the mixture was adjusted to pH 9.0 and diluted with water to make 10 mL. It was confirmed that pH remained unchanged after dilution. The residual rate was measured by the method shown in Test Example 1, and the appearance was examined by the method shown in Test Example 2.

Example 6

To a suspension of 503 mg of Compound A in 5 mL of water, 64 mg of N-ethyldiethanolamine was added, and the mixture was adjusted to pH 9.0 and diluted with water to make 10 mL. It was confirmed that pH remained unchanged after dilution. The residual rate was measured by the method shown in Test Example 1, and the appearance was examined by the method shown in Test Example 2.

Example 7

To a suspension of 503 mg of Compound A in 5 mL of water, 79 mg of bicine was added, and the mixture was adjusted to pH 9.0 and diluted with water to make 10 mL. It was confirmed that pH remained unchanged after dilution. The residual rate was measured by the method shown in Test Example 1, and the appearance was examined by the method shown in Test Example 2.

Example 8

To a suspension of 499 mg of Compound A in 5 mL of water, 84 mg of tricine was added, and the mixture was adjusted to pH 9.0 and diluted with water to make 10 mL. It was confirmed that pH remained unchanged after dilution. The residual rate was measured by the method shown in Test Example 1, and the appearance was examined by the method shown in Test Example 2.

Example 9

To a suspension of 502 mg of Compound A in 5 mL of water, 56 mg of DABCO was added, and the mixture was adjusted to pH 9.0 and diluted with water to make 10 mL. It was confirmed that pH remained unchanged after dilution. The residual rate was measured by the method shown in Test Example 1, and the appearance was examined by the method shown in Test Example 2.

Example 10

To a suspension of 504 mg of Compound A in 5 mL of water, 51 mg of serine was added, and the mixture was adjusted to pH 9.0 and diluted with water to make 10 mL. It was confirmed that pH remained unchanged after dilution. The residual rate was measured by the method shown in Test Example 1, and the appearance was examined by the method shown in Test Example 2.

Example 11

To a suspension of 499 mg of Compound A in 5 mL of water, 58 mg of threonine was added, and the mixture was adjusted to pH 9.0 and diluted with water to make 10 mL. It was confirmed that pH remained unchanged after dilution. The residual rate was measured by the method shown in Test Example 1, and the appearance was examined by the method shown in Test Example 2.

Example 12

To a suspension of 496 mg of Compound A in 5 mL of water, 76 mg of histidine was added, and the mixture was adjusted to pH 9.0 and diluted with water to make 10 mL. It was confirmed that pH remained unchanged after dilution. The residual rate was measured by the method shown in Test Example 1, and the appearance was examined by the method shown in Test Example 2.

Example 13

To a suspension of 503 mg of Compound A in 5 mL of water, 58 mg of valine was added, and the mixture was adjusted to pH 9.0 and diluted with water to make 10 mL. It was confirmed that pH remained unchanged after dilution. The residual rate was measured by the method shown in Test Example 1, and the appearance was examined by the method shown in Test Example 2.

Example 14

To a suspension of 503 mg of Compound A in 5 mL of water, 64 mg of leucine was added, and the mixture was adjusted to pH 9.0 and diluted with water to make 10 mL. It was confirmed that pH remained unchanged after dilution. The residual rate was measured by the method shown in Test Example 1, and the appearance was examined by the method shown in Test Example 2.

Example 15

To a suspension of 504 mg of Compound A in 5 mL of water, 72 mg of glutamic acid was added, and the mixture was adjusted to pH 9.0 and diluted with water to make 10 mL. It was confirmed that pH remained unchanged after dilution. The residual rate was measured by the method shown in Test Example 1, and the appearance was examined by the method shown in Test Example 2.

Example 16

To a suspension of 500 mg of Compound A in 5 mL of water, 73 mg of asparagine monohydrate was added, and the mixture was adjusted to pH 9.0 and diluted with water to make 10 mL. It was confirmed that pH remained unchanged after dilution. The residual rate was measured by the method shown in Test Example 1, and the appearance was examined by the method shown in Test Example 2.

Example 17

To a suspension of 502 mg of Compound A in 5 mL of water, 36 mg of glycine was added, and the mixture was adjusted to pH 9.0 and diluted with water to make 10 mL. It was confirmed that pH remained unchanged after dilution. The residual rate was measured by the method shown in Test Example 1, and the appearance was examined by the method shown in Test Example 2.

Example 18

To a suspension of 503 mg of Compound A in 5 mL of water, 43 mg of alanine was added, and the mixture was adjusted to pH 9.0 and diluted with water to make 10 mL. It was confirmed that pH remained unchanged after dilution. The residual rate was measured by the method shown in Test Example 1, and the appearance was examined by the method shown in Test Example 2.

Example 19

To a suspension of 503 mg of Compound A in 5 mL of water, 177 mg of disodium edetate hydrate was added, and the mixture was adjusted to pH 9.0 and diluted with water to make 10 mL. It was confirmed that pH remained unchanged after dilution. The residual rate was measured by the method shown in Test Example 1, and the appearance was examined by the method shown in Test Example 2.

Example 20

To a suspension of 500 mg of Compound A in 5 mL of water, 93 mg of sodium pyrosulfite was added, and the mixture was adjusted to pH 9.0 and diluted with water to make 10 mL. It was confirmed that pH remained unchanged after dilution. The residual rate was measured by the method shown in Test Example 1, and the appearance was examined by the method shown in Test Example 2.

Example 21

To a suspension of 502 mg of Compound A in 5 mL of water, 117 mg of HEPES was added, and the mixture was adjusted to pH 9.0 and diluted with water to make 10 mL. It was confirmed that pH remained unchanged after dilution. The residual rate was measured by the method shown in Test Example 1, and the appearance was examined by the method shown in Test Example 2.

Example 22

To a suspension of 502 mg of Compound A in 5 mL of water, 64 mg of aspartic acid was added, and the mixture was adjusted to pH 9.0 and diluted with water to make 10 mL. It was confirmed that pH remained unchanged after dilution. The residual rate was measured by the method shown in Test Example 1, and the appearance was examined by the method shown in Test Example 2.

Comparative Example 1

To a suspension of 498 mg of Compound A in 5 mL of water, 1 M sodium hydroxide aqueous solution was added to adjust the mixture to pH 8.9, and the mixture was diluted with water to make 10 mL. It was confirmed that pH remained unchanged after dilution. The residual rate was measured by the method shown in Test Example 1, and the appearance was examined by the method shown in Test Example 2.

Comparative Example 2

To a suspension of 504 mg of Compound A in 5 mL of water, 95 mg of meglumine was added, and the mixture was adjusted to pH 9.0 and diluted with water to make 10 mL. It was confirmed that pH remained unchanged after dilution. The residual rate was measured by the method shown in Test Example 1, and the appearance was examined by the method shown in Test Example 2.

It was confirmed that the stability of Compound A in an aqueous solution had been improved by adding a stabilizing component, as compared with Comparative Examples. In view of appearance, the results with trometamol, bicine, tricine, and EDTA were particularly favorable.

**Claims**

1. A pharmaceutical composition comprising Components (1) and (2) :

    (1) 6-fluoro-3-hydroxy-2-pyrazinecarboxamide or a salt thereof; and
    (2) a compound having a partial structure containing two heteroatoms separated by at least two carbon atoms, or a sulfite thereof.

2. The pharmaceutical composition according to claim 1, wherein Component (2) is at least one of the following Components (2-1) to (2-6):

    (2-1) a hydroxyalkylamine;
    (2-2) a heterocyclic amine;
    (2-3) an α-amino acid with an isoelectric point of 10 or lower;
    (2-4) an aminocarboxylic acid chelating agent;
    (2-5) a β-aminosulfonic acid; and
    (2-6) a sulfite.

3. The pharmaceutical composition according to claim 2, wherein the hydroxyalkylamine of Component (2-1) is represented by the following general formula [1] or [2]:

[Formula 1]

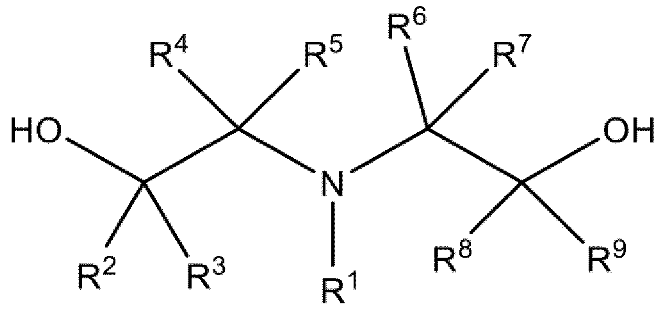

[1]

wherein $R^1$ represents a hydrogen atom or a $C_{1-3}$ alkyl group, wherein the $C_{1-3}$ alkyl group may have a hydroxy group or a carboxy group as a substituent group, and $R^2$ to $R^9$, which are identical to or different from each other, represent a hydrogen atom or a $C_{1-3}$ alkyl group.

[Formula 2]

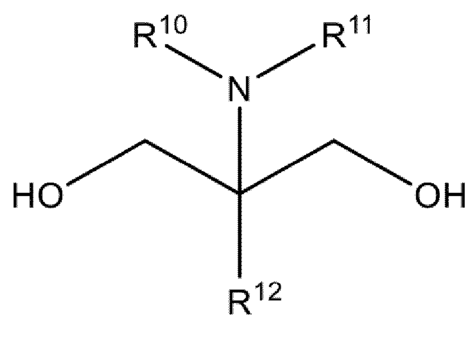

[2]

wherein $R^{10}$ and $R^{11}$, which are identical to or different from each other, represent a hydrogen atom or a $C_{1-3}$ alkyl group, wherein the $C_{1-3}$ alkyl group may have a hydroxy group or a carboxy group as a substituent group, and $R^{12}$ represents a hydrogen atom or a $C_{1-3}$ alkyl group, wherein the $C_{1-3}$ alkyl group may have a hydroxy group as a substituent group.

**4.** The pharmaceutical composition according to claim 2, wherein the hydroxyalkylamine of Component (2-1) is selected from the following compound group:
a compound group consisting of trometamol, diethanolamine, triethanolamine, diisopropanolamine, N-ethyldiethanolamine, bicine, and tricine.

**5.** The pharmaceutical composition according to any of claims 2 to 4, wherein the heterocyclic amine of Component (2-2) is DABCO.

**6.** The pharmaceutical composition according to any of claims 2 to 5, wherein the $\alpha$-amino acid with an isoelectric point of 10 or lower of Component (2-3) is selected from the following compound group:
serine, threonine, histidine, valine, leucine, glutamic acid, glutamine, cysteine, phenylalanine, aspartic acid, asparagine, glycine, alanine, and salts of the amino acids.

**7.** The pharmaceutical composition according to any of claims 2 to 6, wherein the aminocarboxylic acid chelating agent of Component (2-4) is EDTA.

**8.** The pharmaceutical composition according to any of claims 2 to 7, wherein the $\beta$-aminosulfonic acid of Component (2-5) is taurine or HEPES.

**9.** The pharmaceutical composition according to any of claims 2 to 8, wherein the sulfite of Component (2-6) is sodium pyrosulfite.

**10.** The pharmaceutical composition according to any of claims 1 to 9, comprising 0.05 to 5 equivalents of Component (2) to 6-fluoro-3-hydroxy-2-pyrazinecarboxamide or a salt thereof.

**11.** The pharmaceutical composition according to any of claims 1 to 10, which further comprises Component (3) and is an aqueous solution:
(3) water.

**12.** The pharmaceutical composition according to claim 11, wherein the concentration of Component (2) is 0.1 to 5% w/v.

**13.** The pharmaceutical composition according to claim 11 or 12, wherein the aqueous solution is pH 6.7 to 12.5.

**14.** The pharmaceutical composition according to any of claims 1 to 10, which is a lyophilized preparation.

**INTERNATIONAL SEARCH REPORT**

International application No.
**PCT/JP2021/045287**

**A. CLASSIFICATION OF SUBJECT MATTER**

***A61K 31/4965***(2006.01)i; ***A61K 9/08***(2006.01)i; ***A61K 9/19***(2006.01)i; ***A61K 47/04***(2006.01)i; ***A61K 47/18***(2006.01)i; ***A61K 47/20***(2006.01)i; ***A61K 47/22***(2006.01)i; ***A61P 31/12***(2006.01)i
FI: A61K31/4965; A61P31/12; A61K9/08; A61K9/19; A61K47/18; A61K47/22; A61K47/20; A61K47/04

According to International Patent Classification (IPC) or to both national classification and IPC

**B. FIELDS SEARCHED**

Minimum documentation searched (classification system followed by classification symbols)

A61K31/4965; A61K9/08; A61K9/19; A61K47/04; A61K47/18; A61K47/20; A61K47/22; A61P31/12

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | WO 2012/043696 A1 (TOYAMA CHEMICAL CO., LTD.) 05 April 2012 (2012-04-05) claims 4, 7, 8, 11, paragraphs [0024]-[0027], examples 10-13, 30, test example 1 | 1-14 |
| X | WO 2012/043700 A1 (TOYAMA CHEMICAL CO., LTD.) 05 April 2012 (2012-04-05) claims 2, 3, paragraphs [0024]-[0029], examples 13-27, test example 1 | 1-14 |
| A | WO 2018/003946 A1 (TOYAMA CHEMICAL CO., LTD.) 04 January 2018 (2018-01-04) | 1-14 |
| A | WO 2019/131223 A1 (FUJIFILM TOYAMA CHEMICAL CO LTD) 04 July 2019 (2019-07-04) | 1-14 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 February 2022** | **22 February 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2021/045287**

| Patent document cited in search report | Publication date (day/month/year) | Patent family member(s) | Publication date (day/month/year) |
|---|---|---|---|
| WO 2012/043696 A1 | 05 April 2012 | US 2013/0217708 A1<br>claims 4, 7, 8, 11, paragraphs [0099]-[0117], examples 10-13, 30, test example 1 | |
| | | EP 2623497 A1 | |
| | | CN 103209967 A | |
| WO 2012/043700 A1 | 05 April 2012 | US 2013/0274472 A1<br>claims 2, 3, paragraphs [0086]-[0105], examples 13-27, test example 1 | |
| | | EP 2623498 A1 | |
| | | CN 103209966 A | |
| WO 2018/003946 A1 | 04 January 2018 | TW 201808291 A | |
| WO 2019/131223 A1 | 04 July 2019 | US 2021/0059947 A1 | |
| | | EP 3733182 A1 | |
| | | CN 111556752 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0010569 A
- WO 2012043700 A
- WO 2012043696 A